# EUROPÄISCHE PATENTSCHRIFT

(11) **EP 1 685 404 B1**
(45) Veröffentlichungstag und Bekanntmachung des Hinweises auf die Patenterteilung: **11.02.2009**
(21) Anmeldenummer: 04797930.7
(22) Anmeldetag: 16.11.2004
(51) Int. Cl.: G01N 33/543, B03C 1/00

(54) **VERFAHREN UND VORRICHTUNG ZUR VERBESSERTEN REINIGUNG EINER AN PARAMAGNETISCHE MIKROPARTIKEL GEBUNDENEN SUBSTANZ**
METHOD AND DEVICE FOR IMPROVED CLEANING OF A SUBSTANCE BOUND TO PARAMAGNETIC MICROPARTICLES
PROCEDE ET DISPOSITIF DESTINE AU NETTOYAGE AMELIORE D'UNE SUBSTANCE LIEE A DES MICROPARTICULES PARAMAGNETIQUES

(30) Priorität: 20.11.2003 DE 10354351
(43) Veröffentlichungstag der Anmeldung: 02.08.2006
(73) Patentinhaber: November Aktiengesellschaft, 91056 Erlangen (DE)
(72) Erfinder: BARTEN, Roland, 35039 Marburg (DE); KRAMER, Marion, 91301 Forchheim (DE); KRAUSE, Jürgen, 91052 Erlangen (DE); STRICH, Sandra, 91058 Erlangen (DE); WEILAND, Anja, 91099 Poxdorf (DE)
(74) Vertreter: UEXKÜLL & STOLBERG
(86) Internationale Anmeldenummer: PCT/EP2004/012988
(87) Internationale Veröffentlichungsnummer: WO 2005/050208

(56) Entgegenhaltungen:
- WO-A-02/43865
- WO-A-03/049530
- WO-A-20/04096443
- DE-A1- 10 111 520
- US-A1- 2003 095 897

## Beschreibung

Die Erfindung betrifft ein Verfahren zur verbesserten Reinigung einer an paramagnetische Mikropartikel gebundenen Substanz und eine zur Durchführung des Verfahrens geeignete Vorrichtung.

Magnetische Mikropartikel werden vor allem in diagnostischen und analytischen Verfahren eingesetzt. Sie weisen eine im Verhältnis zu ihrer Masse große Oberfläche auf, an der nachzuweisende Analyte über eine Beschichtung spezifisch gebunden werden können. Die Mikropartikel können mit Hilfe eines Magnetfelds reversibel immobilisiert werden. Nicht gebundene Stoffe können dann zusammen mit der diese Stoffe enthaltenden Flüssigkeit von den Mikropartikeln abgesondert werden. Die Mikropartikel können danach in einer Waschflüssigkeit gewaschen und spezifisch gebundene Stoffe mit einer Elutionsflüssigkeit eluiert werden. Ein beim Absondern auftretender Flüssigkeitsstrom kann bewirken, dass ein Teil der magnetisch immobilisierten Mikropartikel suspendiert und mitgeschwemmt wird. Das führt zu einem Verlust der gebundenen Analyte. Beim Eluieren mitgeschwemmte Mikropartikel können wegen ihres Eisengehalts eine nachfolgende Reaktion, wie z. B. eine Polymerase-Kettenreaktion (PCR), stören. Weiterhin können durch die mitgeschwemmten Mikropartikel Detektionsverfahren gestört werden, die auf optischen Eigenschaften beruhen, weil die Mikropartikel Licht streuen und absorbieren können und weil sie eine Fluoreszenz aufweisen können. Darüber hinaus können elektrochemische Detektionsverfahren beeinflusst werden, z. B. weil das in den Mikropartikeln enthaltende Eisen dabei reduziert werden kann. Durch die mitgeschwemmten Mikropartikel kann die Empfindlichkeit des diagnostischen oder analytischen Verfahrens verringert werden.

Bei medizinischen Anwendungen des gereinigten Stoffs können mitgeschwemmte Mikropartikel immunogen wirken oder eine Thrombenbildung induzieren. Ein weiteres bei medizinischen Anwendungen auftretendes Problem besteht darin, dass die Mikropartikel Filter zusetzen können, die beispielsweise zum Sterilfiltrieren von zu verabreichenden Lösungen eingesetzt werden. Nachteilig ist auch, dass die Mikropartikel den gereinigten Stoff wieder binden und dadurch die zur Verfügung stehende Menge dieses Stoffs verringern können.

Aus der US 4,910,148 ist eine Vorrichtung zum Absondern magnetisierter Partikel aus biologischen Flüssigkeiten, in denen die magnetisierten Partikel suspendiert sind, bekannt. Die Vorrichtung enthält eine magnetische Platte mit einer Vielzahl von Permanentmagneten. Zum Absondern der magnetisierten Partikel wird die biologische Flüssigkeit mit den darin suspendierten magnetisierten Partikeln in einem Behälter über der Platte bewegt. Während die magnetisierten Partikel durch die magnetische Platte zurückgehalten werden, wird die biologische Flüssigkeit abgesaugt.

Aus der EP 0 237 549 B1 ist ein Abscheidegerät zum Abtrennen magnetischer Partikel aus einem flüssigen Medium bekannt. Das Abscheidegerät enthält einen Abscheider mit einer Strömungskammer mit einem Eingang und einem Ausgang für das flüssige Medium sowie ein Magnetisierungsmittel. Je nach Stellung der Strömungskammer im Verhältnis zum Magnetisierungsmittel bildet sich in der Strömungskammer ein starkes oder ein schwaches Magnetfeld aus. Das Abscheidegerät kann aus einem ersten und einem zweiten derart aufgebauten Abscheider bestehen. Dadurch kann ein Probendurchsatz erhöht werden. Weiterhin kann eine Fraktionierung einer Probe durch unterschiedlich eingestellte Abscheider erreicht werden.

Aus der US 2003/0095897 A1 ist ein Verfahren bekannt, bei dem eine Flüssigkeit mit darin dispergierten magnetischen Partikeln durch ein magnetisches Feld geleitet wird, wobei die magnetischen Partikel festgehalten werden. Das Magnetfeld ist konstant. Das Lösen der magnetisch festgehaltenen Partikel erfolgt durch einen gepulsten Flüssigkeitsstrom.

Bei allen bekannten Verfahren besteht ein Nachteil darin, dass die Strömung einer Flüssigkeit, die an den magnetisch bereits festgehaltenen Partikeln vorbei strömt, ein Suspendieren dieser Partikel bewirken kann. Dadurch können die bereits magnetisch festgehaltenen Partikel mit der Flüssigkeit mitgeschwemmt werden. Damit sind die oben genannten Nachteile verbunden.

Aufgabe der vorliegenden Erfindung ist es, insbesondere diese Nachteile nach dem Stand der Technik zu beseitigen. Es soll ein Verfahren und eine Vorrichtung bereitgestellt werden, die es erlauben, paramagnetische Mikropartikel in einem fluidischen System möglichst quantitativ magnetisch festzuhalten und gegebenenfalls wieder freizusetzen. Das Verfahren und die Vorrichtung sollen einfach und preiswert realisierbar sein.

Erfindungsgemäß wird die Aufgabe durch die Merkmale der Ansprüche 1 und 13 gelöst. Zweckmäßige Ausgestaltungen ergeben sich aus den Merkmalen der Ansprüche 2 bis 12 und 14 bis 30.

Erfindungsgemäß ist ein Verfahren zur verbesserten Reinigung einer ersten Substanz, welche an paramagnetische Mikropartikel gebunden ist, wobei die Mikropartikel in einer ersten Flüssigkeit suspendiert sind, mit folgenden Schritten vorgesehen:
a) die Mikropartikel werden in einem ersten Behälter einem ersten Magnetfeld ausgesetzt, um sie dadurch festzuhalten und daran zu hindern, mit einem Strom der ersten Flüssigkeit mitgeschwemmt zu werden und
b) zumindest ein Teil der ersten Flüssigkeit wird nach Schritt lit. a in einer ersten Richtung durch eine erste Leitung durch einen Abschnitt der ersten Leitung hindurch geleitet und in dem Abschnitt einem zweiten oder erneut dem ersten Magnetfeld ausgesetzt, um dennoch mitgeschwemmte Mikropartikel dadurch festzuhalten, wobei in dem Abschnitt die Querschnittfläche der ersten Leitung vergrößert ist,
wobei das zweite oder erste Magnetfeld innerhalb des Abschnitts eine größere durchschnittliche Feldstärke aufweist als das erste Magnetfeld innerhalb des ersten Behälters.

Bei der Substanz kann es sich um Zellen, Moleküle oder Aggregate handeln. Bei dem Teil der ersten Flüssigkeit gemäß Schritt lit. b handelt es sich bevorzugt um einen überwiegenden Teil der ersten Flüssigkeit oder um die gesamte erste Flüssigkeit. Wenn die Mikropartikel beim Schritt lit. a dem ersten Magnetfeld ausgesetzt werden, wird dabei ein überwiegender Teil der in der ersten Flüssigkeit suspendierten Mikropartikel im ersten Magnetfeld zurückgehalten. Das Magnetfeld kann mittels eines Elektromagneten oder eines Permanentmagneten bereitgestellt werden. Das Festhalten gemäß Schritt lit. a kann in einem Teil eines Behälters erfolgen, in welchem die Mikropartikel in der Flüssigkeit suspendiert sind. Der Magnet ist dabei bevorzugt so angeordnet, dass die magnetisch festgehaltenen Mikropartikel sich nicht an einer Position ablagern, in welcher sie den Fluss der Flüssigkeit durch die erste Leitung behindern können. Der erste Behälter kann auch eine zum magnetischen Festhalten der Mikropartikel vorgesehene Kammer mit einer Zu- und einer Ableitung für die erste Flüssigkeit sein.

Beim erfindungsgemäßen Verfahren wird beim Schritt lit. b durch die vergrößerte Querschnittfläche der ersten Leitung in dem Abschnitt die Strömungsgeschwindigkeit des Stroms der ersten Flüssigkeit reduziert. Dadurch wird das Festhalten der Mikropartikel im Abschnitt durch das erste oder zweite Magnetfeld erleichtert. Der Abschnitt der Leitung mit der vergrößerten Querschnittfläche kann in Form einer Kammer vorliegen. Das zweite oder erste Magnetfeld innerhalb des Abschnitts kann dadurch eine größere durchschnittliche Feldstärke aufweisen als das erste Magnetfeld innerhalb des ersten Behälters, dass der Abschnitt ein relativ geringes Volumen aufweist und sich dieses Volumen insgesamt dicht an einem das erste oder zweite Magnetfeld bewirkenden Magneten befindet. Somit kann die durchschnittliche Feldstärke im Abschnitt selbst dann größer sein als die durchschnittliche Feldstärke im ersten Behälter, wenn das zweite Magnetfeld insgesamt nicht stärker ist als das erste Magnetfeld oder das erste Magnetfeld sowohl im Behälter als auch im Abschnitt wirkt. Durch das Verfahren können aus dem ersten Behälter weggeschwemmte Mikropartikel im Abschnitt nahezu quantitativ festgehalten werden.

Die Reinigung gemäß dem erfindungsgemäßen Verfahren kann zur Probenaufbereitung für die Durchführung einer PCR oder ein Detektionsverfahren erfolgen. Sie kann auch dazu dienen, die erste Substanz für eine sonstige, beispielsweise medizinische, Anwendung zu reinigen. Bei den Anwendungen können die paramagnetischen Mikropartikel eine unerwünschte Verunreinigung darstellen.

Die im Abschnitt festgehaltenen Mikropartikel werden vorzugsweise zu den im Schritt lit. a festgehaltenen Mikropartikeln zurückgeführt. Dazu kann nach dem Schritt lit. b eine zweite und/oder weitere Flüssigkeit in einer zweiten Richtung in den Abschnitt und in einer der ersten Richtung entgegengesetzten Richtung durch die erste Leitung geleitet werden, wobei ein Einwirken des ersten und des gegebenenfalls vorhandenen zweiten Magnetfelds auf die Mikropartikel aufgehoben wird, so dass die im Abschnitt festgehaltenen Mikropartikel suspendiert und zumindest teilweise zu den beim Schritt lit. a festgehaltenen Mikropartikeln zurückgeschwemmt werden. Dadurch kann der Verlust an paramagnetischen Mikropartikeln bei der Durchführung von Waschschritten deutlich verringert und die Ausbeute an der zu reinigenden ersten Substanz deutlich erhöht werden.

Die zweite Richtung, mit der die zweite und/oder weitere Flüssigkeit in den Abschnitt geleitet wird, kann beispielsweise senkrecht zur ersten Richtung sein, so dass die zweite und/oder weitere Flüssigkeit direkt auf die zurückgehaltenen Mikropartikel strömt und diese suspendiert. Das Abfließen der zweiten und/oder weiteren Flüssigkeit durch die erste Leitung erfolgt in einer der ersten Richtung entgegengesetzten Richtung. Auch die zweite Richtung kann eine der ersten Richtung entgegengesetzte Richtung sein. Das vereinfacht den Aufbau der Vorrichtung, die ansonsten für das Einströmen der zweiten und/oder weiteren Flüssigkeit in den Abschnitt beispielsweise eine weitere Leitung aufweisen müsste.

Die zweite Flüssigkeit ist im Allgemeinen eine Waschlösung. Diese kann eine identische Zusammensetzung aufweisen, wie die erste Flüssigkeit. Die zweite Flüssigkeit wird vorzugsweise so gewählt, dass die erste Substanz darin an die Mikropartikel gebunden bleibt und weitere Substanzen oder sonstige Verunreinigungen sich von den Mikropartikeln oder der ersten Substanz lösen. Das Lösen kann z. B. dadurch erreicht werden, dass die zweite Flüssigkeit ein Detergenz enthält. Die weitere Flüssigkeit kann so gewählt werden, dass sich die erste Substanz darin von den Mikropartikeln löst. Es handelt sich dabei im Allgemeinen um eine Elutionslösung.

Der Abschnitt ist vorzugsweise so geformt, dass beim Strömen der zweiten und/oder weiteren Flüssigkeit in der zweiten Richtung im Abschnitt Verwirbelungen entstehen, so dass dort abgelagerte Mikropartikel suspendiert werden. Das kann z.B. durch einen entsprechend im Abschnitt angeordneten Vorsprung oder eine sonstige Einrichtung zum Leiten eines Flüssigkeitsstroms erfolgen. Besonders bevorzugt ist es, wenn eine zweite Leitung in dem Abschnitt eine Öffnung aufweist, über welche die zweite und/oder weitere Flüssigkeit so in den Abschnitt geleitet wird, dass im Abschnitt Verwirbelungen entstehen und dort abgelagerte Mikropartikel suspendiert werden.

Die Schritte lit. a und lit. b können mit der zweiten und/oder weiteren Flüssigkeit anstatt der ersten Flüssigkeit wiederholt werden. Auch dabei werden die Mikropartikel weit gehend aus der zweiten und/oder weiteren Flüssigkeit entfernt. Ist die weitere Flüssigkeit eine Elutionsflüssigkeit, kann diese nach Schritt lit. b direkt für ein Nachweisverfahren, wie eine PCR, eingesetzt werden. Das Nachweisverfahren wird durch die Mikropartikel nicht oder kaum beeinträchtigt, da diese, zumindest nahezu, quantitativ zurückgehalten werden. Ein Entfernen noch in der weiteren Flüssigkeit enthaltener Mikropartikel, beispielsweise mittels eines Filters, ist nicht erforderlich. Das vereinfacht die Automatisierung des Verfahrens, da beim Filtrieren stets die Gefahr des Zusetzens des Filters bestehen würde und der zugesetzte Filter dann üblicherweise manuell ausgetauscht werden müsste.

Das erste Magnetfeld kann in einem Bereich innerhalb des ersten Behälters wirken. Die Mikropartikel können dem ersten Magnetfeld ausgesetzt werden, indem ein Permanentmagnet an den Bereich und den Abschnitt heran geführt wird. Mit einem einzigen Magneten kann ein Magnetfeld erzeugt werden, bei welchem beispielsweise im Bereich vor allem der eine Pol des Magneten und im Abschnitt vor allem der andere Pol des Magneten ein starkes Magnetfeld verursacht. Die Mikropartikel können dem ersten und zweiten Magnetfeld auch ausgesetzt werden, indem jeweils ein Permanentmagnet an den Bereich und den Abschnitt heran geführt wird.

Die Mikropartikel weisen bevorzugt einen mittleren Durchmesser von 50 nm bis 50 µm, vorzugsweise von 500 nm bis 50 µm, auf. Solche Mikropartikel haben sich als besonders günstig für die Reinigung von Substanzen erwiesen. Die Mikropartikel können eine Beschichtung aus Glas, Silikat, Silan, einem Ionenaustauscher, einem Rezeptor, einem Liganden, einem Antigen, einem Antikörper oder einer Nukleinsäure aufweisen.

Weiterhin betrifft die Erfindung eine Vorrichtung gemäß Anspruch 13 zur Durchführung eines erfindungsgemäßen Verfahrens.

Der Bereich des ersten Behälters ist ein Gebiet innerhalb des ersten Behälters und bevorzugt in der Nähe der Mündung der ersten Leitung im ersten Behälter. Der Bereich ist vorzugsweise so angeordnet, dass darin magnetisch festgehaltene paramagnetische Mikropartikel einen Flüssigkeitsfluss durch die erste Leitung nicht behindern.

Der Magnet kann fest mit der Vorrichtung verbunden und beispielsweise als bei Bedarf aktivierbarer Elektromagnet ausgebildet sein.

Besonders bevorzugt sind der Bereich, der Abschnitt und die erste Aussparung oder der erste Magnet so angeordnet, dass ein vom ersten Magneten erzeugtes oder erzeugbares Magnetfeld sowohl im Bereich als auch im Abschnitt wirken kann oder wirkt. Das kann beispielsweise dadurch gewährleistet werden, dass der Bereich und der Abschnitt sich auf gegenüberliegenden Seiten der ersten Aussparung befinden, in die der Magnet eingeführt werden kann, so dass ein Pol des Magneten auf den Bereich und der andere Pol des Magneten auf den Abschnitt wirken kann.

Bevorzugt ist der Magnet ein Permanentmagnet. Zur Einwirkung auf die Mikropartikel kann er an den Abschnitt oder den Bereich herangeführt werden. Das ist bei einer automatisierten Probenaufbereitung einfach zu gewährleisten. Die Vorrichtung selbst muss dann keinen Magneten aufweisen.

Der Abschnitt kann als eine Aussparung in der ersten Leitung ausgebildet sein. Die Aussparung kann dabei breit und flach ausgebildet sein. Dadurch kann eine hohe magnetische Feldstärke im Bereich der Aussparung bereitgestellt werden. Weiterhin kann dadurch die Strömungsgeschwindigkeit verlangsamt werden. Die Aussparung ist vorzugsweise so bemessen, dass sich die Mikropartikel darin ablagern können, ohne dadurch die Querschnittfläche der ersten Leitung im Bereich der Aussparung gegenüber der sonstigen Querschnittfläche der ersten Leitung zu verkleinern. Ein solches Verkleinern der Querschnittfläche würde eine Verengung in der ersten Leitung und dadurch eine Erhöhung der Strömungsgeschwindigkeit bewirken. Das könnte dazu führen, dass abgelagerte Mikropartikel mitgeschwemmt werden.

Der Abschnitt ist vorzugsweise so geformt, dass beim Strömen einer Flüssigkeit in eine erste Richtung im Abschnitt eine laminare Strömung und beim Strömen der Flüssigkeit in eine zweite, insbesondere der ersten Richtung entgegengesetzte, Richtung eine verwirbelte Strömung entstehen kann. Dadurch können die im Abschnitt abgelagerten Mikropartikel besonders effizient suspendiert und zu den im Bereich abgelagerten Mikropartikeln zurückgeführt werden.

Vorzugsweise zweigt von der ersten Leitung mindestens eine zweite Leitung ab. Beispielsweise kann sich die erste Leitung dazu in zwei Leitungen aufgabeln. Die Abzweigung bzw. Gabelung kann sich im Abschnitt oder in Strömungsrichtung einer vom ersten Behälter durch die erste Leitung strömenden Flüssigkeit nach dem Abschnitt befinden. Eine Öffnung der zweiten Leitung kann im Abschnitt münden, wobei die Öffnung so angeordnet ist, dass durch die Öffnung in den Abschnitt strömende Flüssigkeit im Abschnitt Verwirbelungen verursachen kann und dadurch dort abgelagerte Mikropartikel suspendiert werden können.

Als besonders günstig hat es sich erwiesen, wenn die erste Leitung einen Durchmesser von 50 µm bis 2 mm, bevorzugt von 100 µm bis 500 µm, aufweist. Vorteilhafterweise weist der Abschnitt eine Querschnittfläche auf, die höchstens dreimal, vorzugsweise höchstens zweimal, so groß ist, wie die Querschnittfläche der ersten Leitung. Das ermöglicht eine ausreichende Verringerung der Strömungsgeschwindigkeit, um eine Ablagerung der Mikropartikel im ersten oder zweiten Magnetfeld zu ermöglichen. Vorzugsweise weist der Abschnitt eine Querschnittfläche von höchstens 2 mm², vorzugsweise 1 mm², auf.

In der erfindungsgemäßen Vorrichtung kann ein zweiter Behälter zur Bereitstellung einer zweiten Flüssigkeit, ein dritter Behälter zur Bereitstellung einer weiteren Flüssigkeit und/oder ein vierter Behälter zur Aufnahme der ersten und ggf. zweiten und/oder weiteren Flüssigkeit vorgesehen sein. Dadurch ist es möglich, die Vorrichtung als geschlossenes System bereitzustellen und die erste, zweite und/oder weitere Flüssigkeit nur innerhalb der Vorrichtung zu bewegen, ohne dass in der Vorrichtung enthaltene Flüssigkeit nach außen dringen kann. Das ist insbesondere dann vorteilhaft, wenn mittels der Vorrichtung ein infektiöses oder sonst zumindestpotenziell gefährliches Material, wie beispielsweise Blut, zur Reinigung der Substanzen bearbeitet werden soll. Die zweite Leitung kann in den zweiten Behälter münden. Gegebenenfalls vorhandene weitere Leitungen können in den dritten oder vierten Behälter münden.

Im ersten, zweiten, dritten und/oder vierten Behälter kann jeweils ein darin verschiebbarer Kolben vorgesehen sein, mittels dem die erste, zweite oder weitere Flüssigkeit bewegt werden kann. Auch diese Maßnahme ermöglicht es, die Vorrichtung als geschlossenes System bereitzustellen. Zum Bewegen der Flüssigkeiten in der Vorrichtung ist es dann von außen nur erforderlich, die Kolben zu bewegen. Der erste, zweite, dritte und/oder vierte Behälter kann jeweils in Form einer austauschbaren, insbesondere flüssigkeitsgefüllten, Patrone vorgesehen sein. Dadurch können auf einfache Weise verschiedene erste, zweite oder weitere Flüssigkeiten zum Einsatz in der Vorrichtung bereitgestellt werden. Darüber hinaus kann durch den Einsatz von Patronen vermieden werden, dass die Flüssigkeiten offen gehandhabt werden müssen. Auch diese Maßnahme ist daher im Hinblick auf die Bereitstellung einer als geschlossenes System ausgebildeten Vorrichtung vorteilhaft. Statt des ersten, zweiten, dritten oder vierten Behälters kann die Vorrichtung jeweils eine Ausnehmung zur Aufnahme der Patrone aufweisen.

Bevorzugt ist der erste, zweite, dritte und/oder vierte Behälter zylinderförmig ausgebildet. Das hat sowohl im Hinblick auf den Einsatz von Patronen, als auch im Hinblick auf das Bewegen der Flüssigkeit mittels Kolben den Vorteil, dass auf Grund der Rotationssymmetrie ein fehlerhaft orientiertes Einsetzen der Patrone oder des Kolbens weit gehend ausgeschlossen werden kann. Der ersten, zweite, dritte oder vierte Behälter weist vorzugsweise ein maximales Volumen von 50 µl bis 50 ml, bevorzugt von 500 µl bis 5 ml, auf. Enthält der Behälter einen Kolben, bezieht sich das maximale Volumen auf das Volumen bei ausgefahrenem Kolben.

Bevorzugt ist die Vorrichtung in ein Gerät zur, insbesondere automatisierten, Probenprozessierung einsetzbar. Das Gerät und die Vorrichtung sind dabei aufeinander abgestimmt. Das Gerät kann beispielsweise mindestens eine Einrichtung zur Verschiebung des Kolbens bzw. der Kolben in der Vorrichtung aufweisen. Die Vorrichtung kann auch so ausgebildet sein, dass sie flüssigkeitsleitend mit dem Gerät verbunden werden kann. Dadurch kann eine Probe, welche eine zu reinigende Substanz enthält, der Vorrichtung automatisiert von dem Gerät zugeführt werden. Weiterhin kann die gereinigte Substanz in dem Gerät weiterverarbeitet werden.

Besonders preiswert kann die Vorrichtung aus einem Kunststoff, insbesondere Polycarbonat, vorzugsweise mittels eines Spritzguss-Verfahrens, hergestellt werden.

Nachfolgend wird die Erfindung anhand von Zeichnungen näher erläutert. Es zeigen:
- Fig. 1: eine schematische Darstellung einer erfindungsgemäßen Vorrichtung bei einem ersten Arbeitsschritt,
- Fig. 2: eine schematische Darstellung einer erfindungsgemäßen Vorrichtung am Ende eines den Schritten lit. a und b des erfindungsgemäßen Verfahrens entsprechenden zweiten Arbeitsschritts,
- Fig. 3: eine schematische Darstellung einer erfindungsgemäßen Vorrichtung am Ende eines im Anspruch 2 spezifizierten dritten Arbeitsschritts des erfindungsgemäßen Verfahrens,
- Fig. 4: eine Draufsicht auf eine schematische Darstellung eines Abschnitts der ersten Leitung, welcher eine vergrößerte Querschnittfläche aufweist,
- Fig. 5: einen Querschnitt durch diesen Abschnitt der ersten Leitung mit einem Magneten und in dem Abschnitt durch das Magnetfeld des Magneten festgehaltenen paramagnetischen Mikropartikeln und
- Fig. 6: eine schematische Darstellung einer weiteren Ausgestaltung der erfindungsgemäßen Vorrichtung zum Einsatz von ersten und zweiten Behältern in Form von Patronen.

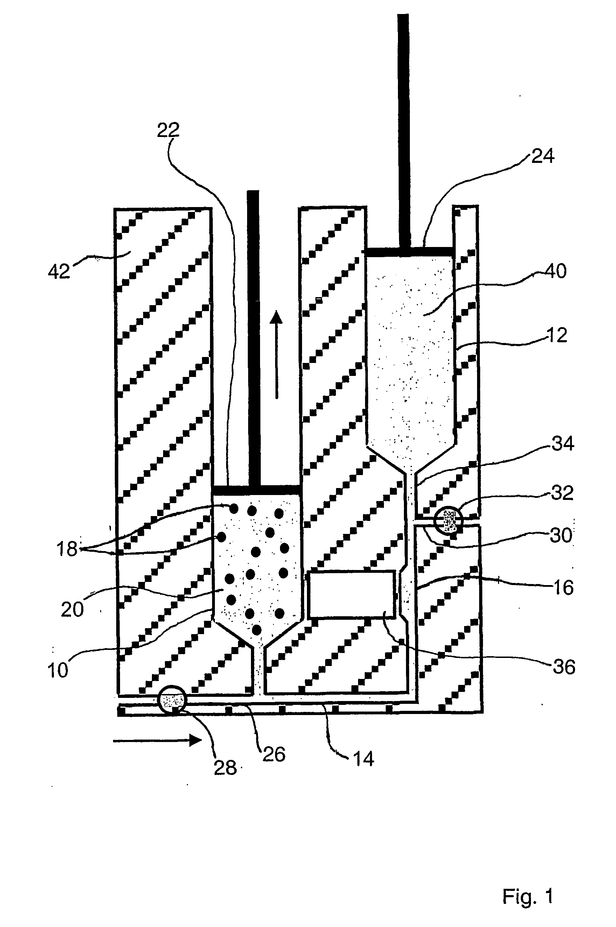

Fig. 1 zeigt eine erfindungsgemäße in einem Substrat 42 gebildete Vorrichtung mit einem ersten Behälter 10, einem zweiten Behälter 12, einer ersten Leitung 14 und einem Abschnitt 16 mit einer gegenüber der ersten Leitung 14 vergrößerten Querschnittfläche. Im ersten Behälter 10 befinden sich paramagnetische Mikropartikel 18, welche in einer ersten Flüssigkeit 20 suspendiert sind. Im ersten 10 und zweiten Behälter 12 befindet sich jeweils ein verschiebbarer Kolben 22, 24. In einer zur ersten Leitung 14 hinführenden Zuleitung 26 befindet sich ein hier in Durchgangsstellung befindliches erstes Ventil 28. In einer vom Abschnitt 16 wegführenden weiteren Leitung 30 befindet sich ein hier in geschlossener Stellung befindliches zweites Ventil 32. Zwischen dem Abschnitt 16 und dem zweiten Behälter 12 befindet sich eine zweite Leitung 34. Zwischen dem Abschnitt 16 und dem ersten Behälter 10 befindet sich eine erste Aussparung 36. Zur Durchführung des Verfahrens wird zunächst der erste Kolben 22 angehoben. Dabei wird Flüssigkeit aus der Zuleitung 26 in den ersten Behälter 10 gesaugt und suspendiert dabei die darin befindlichen magnetischen Mikropartikel 18.

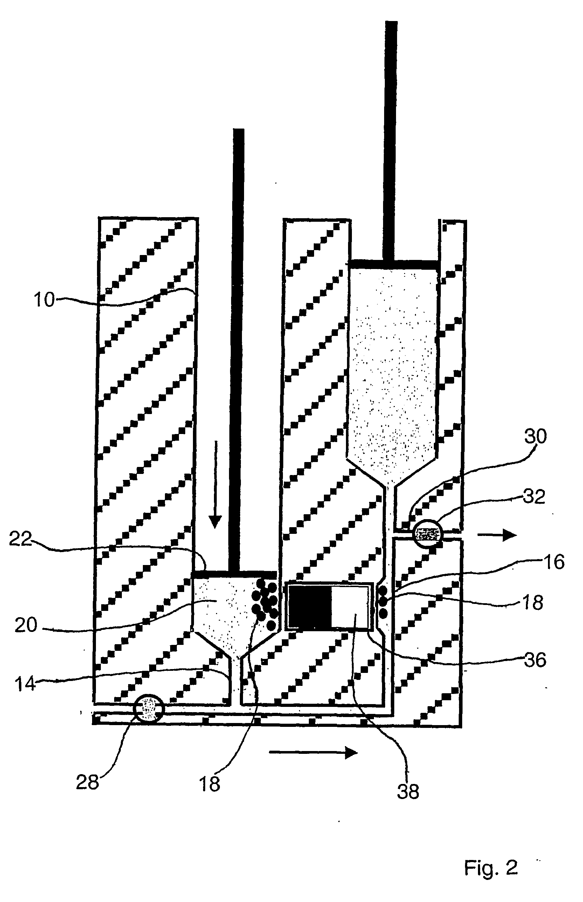

Bei dem in Fig. 2 dargestellten zweiten Arbeitsschritt wird zunächst ein Magnet 38 in die Aussparung 36 eingeführt. Das erste Ventil 28 wird geschlossen und das zweite Ventil 32 geöffnet. Anschließend wird der Kolben 22 nach unten bewegt und dadurch die erste Flüssigkeit 20 durch die erste Leitung 14, den Abschnitt 16 und die weitere Leitung 30 durch das geöffnete zweite Ventil 32 aus dem ersten Behälter 10 herausgedrückt. Dabei werden die magnetischen Mikropartikel 18 in einem Bereich des Behälters und im Abschnitt 16 durch ein vom Magneten 38 erzeugtes erstes Magnetfeld festgehalten. Das erste Magnetfeld weist dabei innerhalb des Abschnitts 16 eine größere durchschnittliche Feldstärke auf als innerhalb des ersten Behälters 10. Das liegt daran, dass der Abschnitt 16 ein kleineres insgesamt näher am Magneten 38 angeordnetes Volumen aufweist, als der Behälter 10. Dadurch ist der durchschnittliche Abstand, den ein Mikropartikel zum Magneten im Abschnitt 16 aufweisen kann, geringer als der durchschnittliche Abstand, den ein Mikropartikel im Behälter 10 aufweisen kann.

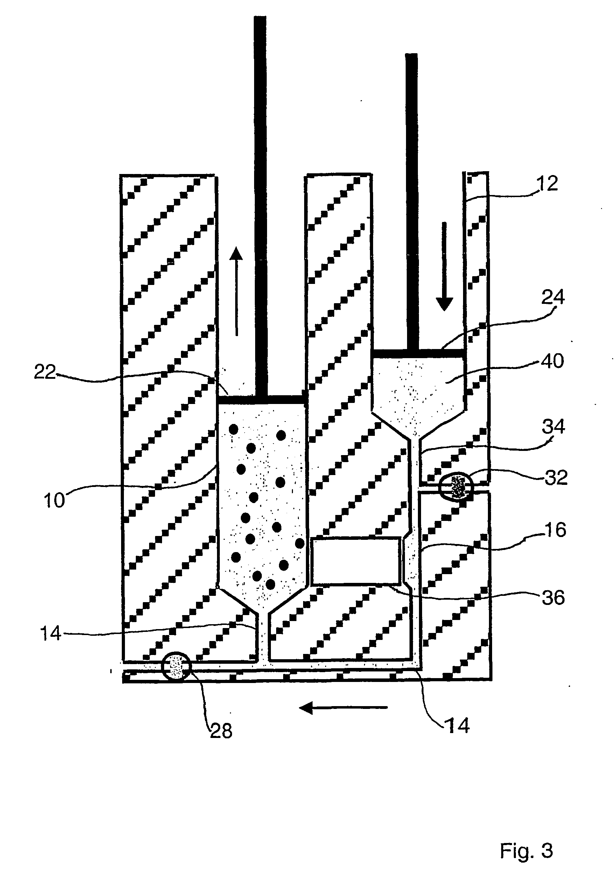

Bei einem dritten in Fig. 3 dargestellten Arbeitsschritt ist der Magnet 38 aus der Aussparung 36 herausgenommen, so dass die paramagnetischen Mikropartikel 18 nicht mehr festgehalten werden. Das zweite Ventil 32 wird geschlossen. Die im zweiten Behälter 12 befindliche zweite Flüssigkeit 40 wird durch Herunterdrücken des Kolbens 24 durch die zweite Leitung 34, den Abschnitt 16 und die erste Leitung 14 in den ersten Behälter 10 gedrückt. Dabei wird der Kolben 22 angehoben. Die paramagnetischen Mikropartikel 18 werden dabei suspendiert. Die im Abschnitt 16 festgehaltenen paramagnetischen Mikropartikel 18 werden dabei in den Behälter 10 zurückgeschwemmt.

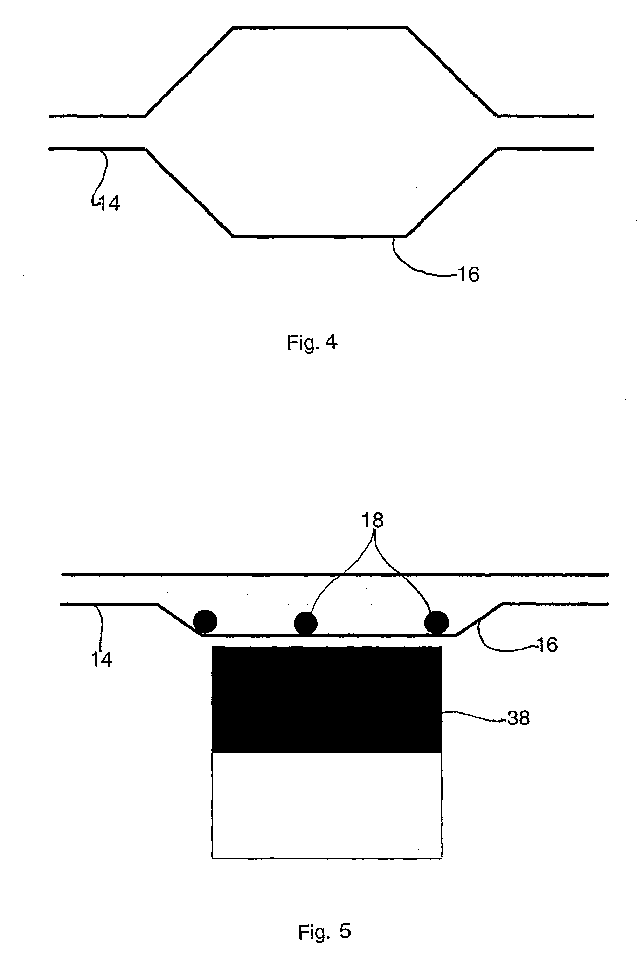

Fig. 4 zeigt eine Draufsicht auf den Abschnitt 16 der ersten Leitung 14, welcher gegenüber der ersten Leitung 14 eine vergrößerte Querschnittfläche aufweist.

Fig. 5 zeigt diesen Abschnitt 16 im Querschnitt. Im Abschnitt 16 befinden sich vom Magnetfeld des Magneten 38 festgehaltene paramagnetische Mikropartikel 18.

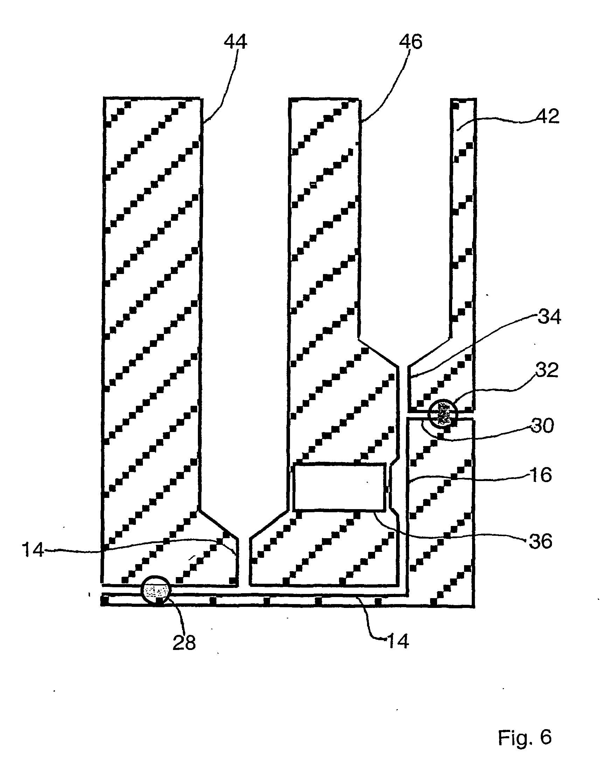

Fig. 6 zeigt eine schematische Darstellung einer weiteren Ausgestaltung der erfindungsgemäßen Vorrichtung. Diese besteht aus einem Substrat 42, in welchem eine erste Aussparung 36 zur Aufnahme eines Magneten sowie eine dritte 44 und eine vierte Aussparung 46 zur Aufnahme jeweils einer den ersten 10 und zweiten Behälter 12 bildenden ersten und zweiten Patrone vorgesehen sind. Die Patronen können jeweils einen Kolben enthalten. Weiterhin weist die Vorrichtung ein erstes Ventil 28 und ein zweites Ventil 32, eine erste Leitung 14, einen Abschnitt 16, eine zweite Leitung 34 und eine weitere Leitung 30 auf. Das Substrat 42 kann aus einem, insbesondere mittels eines Spritzguss-Verfahrens verarbeiteten, Kunststoff bestehen.

### Bezugszeichenliste

- 10: erster Behälter
- 12: zweiter Behälter
- 14: erste Leitung
- 16: Abschnitt
- 18: paramagnetische Mikropartikel
- 20: erste Flüssigkeit
- 22, 24: Kolben
- 26: Zuleitung
- 28: erstes Ventil
- 30: weitere Leitung
- 32: zweites Ventil
- 34: zweite Leitung
- 36: erste Aussparung
- 38: Magnet
- 40: zweite Flüssigkeit
- 42: Substrat
- 44: dritte Aussparung
- 46: vierte Aussparung

## Patentansprüche

1. Verfahren zur verbesserten Reinigung einer ersten Substanz, welche an paramagnetische Mikropartikel (18) gebunden ist, wobei die Mikropartikel (18) in einer ersten Flüssigkeit (20) suspendiert sind, mit folgenden Schritten:
a) die Mikropartikel (18) werden in einem ersten Behälter (10) einem ersten Magnetfeld ausgesetzt, um sie dadurch festzuhalten und daran zu hindern, mit einem Strom der ersten Flüssigkeit (20) mitgeschwemmt zu werden und
b) zumindest ein Teil der ersten Flüssigkeit (20) wird nach Schritt lit. a in einer ersten Richtung durch eine erste Leitung (14) durch einen Abschnitt (16) der ersten Leitung (14) hindurch geleitet und in dem Abschnitt (16) einem zweiten oder dem ersten Magnetfeld ausgesetzt, um dennoch mitgeschwemmte Mikropartikel (18) dadurch festzuhalten, wobei in dem Abschnitt (16) die Querschnittfläche der ersten Leitung (14) vergrößert ist,
wobei das zweite oder erste Magnetfeld innerhalb des Abschnitts (16) eine größere durchschnittliche Feldstärke aufweist als das erste Magnetfeld innerhalb des ersten Behälters (10) .

2. Verfahren nach Anspruch 1, wobei nach dem Schritt lit. b eine zweite (40) und/oder weitere Flüssigkeit in einer zweiten Richtung in den Abschnitt (16) und in einer der ersten Richtung entgegengesetzten Richtung durch die erste Leitung (14) geleitet wird, wobei ein Einwirken des ersten und des gegebenenfalls vorhandenen zweiten Magnetfelds auf die Mikropartikel (18) aufgehoben wird, so dass die im Abschnitt (16) festgehaltenen Mikropartikel (18) suspendiert und zumindest teilweise zu den beim Schritt lit. a festgehaltenen Mikropartikeln (18) zurückgeschwemmt werden.

3. Verfahren nach Anspruch 2, wobei die zweite Richtung der ersten Richtung entgegengesetzt ist.

4. Verfahren nach einem der vorherigen Ansprüche, wobei die zweite Flüssigkeit (40) so gewählt wird, dass die erste Substanz darin an die Mikropartikel (18) gebunden bleibt und weitere Substanzen oder sonstige Verunreinigungen sich von den Mikropartikeln (18) oder der ersten Substanz lösen.

5. Verfahren nach einem der vorherigen Ansprüche, wobei die weitere Flüssigkeit so gewählt wird, dass sich die erste Substanz darin von den Mikropartikeln (18) löst.

6. Verfahren nach einem der vorherigen Ansprüche, wobei der Abschnitt (16) so geformt ist, dass beim Strömen der zweiten (40) und/oder weiteren Flüssigkeit in der zweiten Richtung im Abschnitt Verwirbelungen entstehen, so dass dort abgelagerte Mikropartikel (18) suspendiert werden.

7. Verfahren nach einem der vorherigen Ansprüche, wobei eine zweite Leitung (34) in dem Abschnitt (16) eine Öffnung aufweist, über welche die zweite (40) und/oder weitere Flüssigkeit so in den Abschnitt (16) geleitet wird, dass im Abschnitt (16) Verwirbelungen entstehen und dort abgelagerte Mikropartikel (18) suspendiert werden.

8. Verfahren nach einem der vorherigen Ansprüche, wobei die Schritte lit. a und b mit der zweiten (40) und/oder weiteren Flüssigkeit anstatt der ersten Flüssigkeit (20) wiederholt werden.

9. Verfahren nach einem der vorherigen Ansprüche, wobei das erste Magnetfeld in einem Bereich innerhalb des ersten Behälters (10) wirkt und die Mikropartikel (18) dem ersten Magnetfeld ausgesetzt werden, indem ein Permanentmagnet (38) an den Bereich und den Abschnitt (16) heran geführt wird.

10. Verfahren nach einem der Ansprüche 1 bis 8, wobei das erste Magnetfeld in einem Bereich innerhalb des ersten Behälters (10) wirkt und die Mikropartikel (18) dem ersten und zweiten Magnetfeld ausgesetzt werden, indem jeweils ein Permanentmagnet (38) an den Bereich und den Abschnitt (16) heran geführt wird.

11. Verfahren nach einem der vorherigen Ansprüche, wobei die Mikropartikel (18) einen mittleren Durchmesser von 50 nm bis 50 µm, vorzugsweise von 500 nm bis 50 µm, aufweisen.

12. Verfahren nach einem der vorherigen Ansprüche, wobei die Mikropartikel (18) eine Beschichtung aus Glas, Silikat, Silan, einem Ionenaustauscher, einem Rezeptor, einem Liganden, einem Antigen, einem Antikörper oder einer Nukleinsäure aufweisen.

13. Vorrichtung zur Durchführung eines Verfahrens nach einem der Ansprüche 1 bis 12, enthaltend:
- einen ersten Behälter (10) zur Bereitstellung oder Aufnahme einer ersten Flüssigkeit (20) und von paramagnetischen Mikropartikeln (18),
- eine in den ersten Behälter (10) mündende erste Leitung (14) und
- einen Abschnitt (16) der ersten Leitung (14), welcher gegenüber der sonstigen Querschnittfläche der ersten Leitung (14) eine vergrößerte Querschnittfläche aufweist,
- einen ersten Magneten (38) zur Erzeugung eines ersten Magnetfelds in einem Bereich des ersten Behälters (10) und im Abschnitt (16), wobei der Bereich, der Abschnitt (16) und der erste Magnet (38) so angeordnet sind, dass ein vom ersten Magneten (38) erzeugtes oder erzeugbares Magnetfeld sowohl im Bereich als auch im Abschnitt (16) wirken kann oder wirkt oder
- eine Aussparung (36) zur Aufnahme eines ersten Magneten (38) zur Erzeugung eines ersten Magnetfelds in einem Bereich des ersten Behälters (10) und im Abschnitt (16), wobei der Bereich, der Abschnitt (16) und die Aussparung (36) so angeordnet sind, dass ein vom ersten Magneten (38) erzeugtes oder erzeugbares Magnetfeld sowohl im Bereich als auch im Abschnitt wirken kann oder wirkt, wobei der Bereich und der Abschnitt (16) sich auf gegenüberliegenden Seiten der Aussparung (36) befinden, so dass ein Pol des ersten Magneten (38) auf den Bereich und der andere Pol des ersten Magneten (38) auf den Abschnitt wirken kann oder
- einen ersten Magneten (38) zur Erzeugung eines ersten Magnetfelds in einem Bereich des ersten Behälters (10) und einen zweiten Magneten zur Erzeugung eines zweiten Magnetfelds im Abschnitt (16),
- wobei der Bereich, der Abschnitt (16) und die Aussparung (36) oder der erste Magnet (38) und sofern vorhanden der zweite Magnet so angeordnet und/oder geformt sind, dass das Magnetfeld innerhalb des Abschnitts (16) eine größere durchschnittliche Feldstärke aufweist als das Magnetfeld innerhalb des ersten Behälters (10).

14. Vorrichtung nach Anspruch 13, wobei der Magnet (38) ein Permanentmagnet ist.

15. Vorrichtung nach Anspruch 13 oder 14, wobei der Abschnitt (16) als eine Aussparung in der ersten Leitung (14) ausgebildet ist.

16. Vorrichtung nach einem der Ansprüche 13 bis 15, wobei der Abschnitt (16) so geformt ist, dass beim Strömen einer Flüssigkeit in eine erste Richtung im Abschnitt (16) eine laminare Strömung und beim Strömen der Flüssigkeit in eine zweite, insbesondere der ersten Richtung entgegengesetzte, Richtung eine verwirbelte Strömung entstehen kann.

17. Vorrichtung nach einem der Ansprüche 13 bis 16, wobei von der ersten Leitung (14) mindestens eine zweite Leitung (34) abzweigt.

18. Vorrichtung nach einem der Ansprüche 13 bis 17, wobei eine Öffnung der zweiten Leitung (34) im Abschnitt (16) mündet, wobei die Öffnung so angeordnet ist, dass durch die Öffnung in den Abschnitt (16) strömende Flüssigkeit im Abschnitt (16) Verwirbelungen verursachen kann und dadurch dort abgelagerte Mikropartikel (18) suspendiert werden können.

19. Vorrichtung nach einem der Ansprüche 13 bis 18, wobei die erste Leitung (14) einen Durchmesser von 50 µm bis 2 mm, bevorzugt von 100 µm bis 500 µm, aufweist.

20. Vorrichtung nach einem der Ansprüche 13 bis 19, wobei der Abschnitt (16) eine Querschnittfläche aufweist, die höchstens dreimal, vorzugsweise höchstens zweimal, so groß ist wie die Querschnittfläche der ersten Leitung (14).

21. Vorrichtung nach einem der Ansprüche 13 bis 20, wobei der Abschnitt (16) eine Querschnittfläche von höchstens 2 mm², vorzugsweise höchstens 1 mm², aufweist.

22. Vorrichtung nach einem der Ansprüche 13 bis 21, wobei darin ein zweiter Behälter (12) zur Bereitstellung einer zweiten Flüssigkeit (40), ein dritter Behälter zur Bereitstellung einer weiteren Flüssigkeit und/oder ein vierter Behälter zur Aufnahme der ersten (20) und ggf. zweiten (40) und/oder weiteren Flüssigkeit vorgesehen ist.

23. Vorrichtung nach einem der Ansprüche 13 bis 22, wobei die zweite Leitung (34) in den zweiten Behälter (12) mündet und ggf. vorhandene weitere Leitungen (30) in den dritten oder vierten Behälter münden.

24. Vorrichtung nach einem der Ansprüche 13 bis 23, wobei im ersten (10), zweiten (12), dritten und/oder vierten Behälter jeweils ein darin verschiebbarer Kolben (22, 24) vorgesehen ist, mittels dem die erste (20), zweite (40) oder weitere Flüssigkeit bewegt werden kann.

25. Vorrichtung nach einem der Ansprüche 13 bis 24, wobei der erste (10), zweite (12), dritte und/oder vierte Behälter jeweils in Form einer austauschbaren Patrone (48, 50) vorgesehen ist.

26. Vorrichtung nach einem der Ansprüche 13 bis 25, wobei der erste (10), zweite (12), dritte und/oder vierte Behälter zylinderförmig ausgebildet ist.

27. Vorrichtung nach einem der Ansprüche 13 bis 26, wobei der erste (10), zweite (12), dritte oder vierte Behälter ein maximales Volumen von 50 µl bis 50 ml, bevorzugt von 500 µl bis 5 ml, aufweist.

28. Vorrichtung nach einem der Ansprüche 13 bis 27, wobei die Vorrichtung in ein Gerät zur, insbesondere automatisierten, Probenprozessierung einsetzbar ist.

29. Vorrichtung nach Anspruch 28, wobei das Gerät mindestens eine Einrichtung zur Verschiebung des Kolbens (22, 24) aufweist.

30. Vorrichtung nach einem der Ansprüche 13 bis 29, wobei die Vorrichtung aus einem Kunststoff, insbesondere Polycarbonat, vorzugsweise mittels eines Spritzguss-Verfahrens, hergestellt ist.

## Claims

1. A method for improved purification of a first substance which is bound to paramagnetic microparticles (18), the microparticles (18) being suspended in a first liquid (20), comprising the following steps:
a) the microparticles (18) are exposed in a first container (10) to a first magnetic field, to thereby arrest them and prevent them from being washed away with a stream of the first liquid (20), and
b) after step a), at least part of the first liquid (20) is passed in a first direction through a first line (14), through a portion (16) of the first line (14), and exposed in the portion (16) to a second magnetic field or to the first magnetic field, to thereby arrest microparticles (18) that h nevertheless been washed away, wherein the cross-sectional area of the first line (14) is enlarged in the portion (16),
wherein the second or first magnetic field within the portion (16) has a higher average field strength than the first magnetic filed within the first container (10).

2. The method according to claim 1, wherein after step b) a second (40) and/or further liquid is passed in a second direction into the portion (16) and in a direction opposite to the first direction through the first line (14), wherein an effect of the first and an optionally present second magnetic field on the microparticles (18) is suspended, so that the microparticles (18) arrested in the portion (16) are suspended and at least partially washed back to the microparticles (18) arrested in step a).

3. The method according to claim 2, wherein the second direction is opposite to the first direction.

4. The method according to one of the preceding claims, wherein the second liquid (40) is chosen such that the first substance therein remains bound to the microparticles (18) and further substances or other contaminants are detached from the microparticles (18) or the first substance.

5. The method according to one of the preceding claims, wherein the further liquid is chosen such that the first substance therein is detaching from the microparticles (18).

6. The method according to one of the preceding claims, wherein the portion (16) is shaped such that, when the second (40) and/or further liquid flows in the second direction, turbulences are created in the portion, so that microparticles (18) deposited therein are suspended.

7. The method according to any of the preceding claims, wherein a second line (34) in the portion (16) has an opening via which the second (40) and/or further liquid is passed into the portion (16) in such a way that turbulences are generated in the portion (16) and microparticles (18) deposited therein are suspended.

8. The method according to any of the preceding claims, wherein the steps a) and b) are repeated with the second (40) and/or further liquid instead of the first liquid 20).

9. The method according to any of the preceding claims, wherein the first magnetic field is acting in a region within the first container (10) and the microparticles (18) are exposed to the first magnetic field by a permanent magnet (38) being brought up to the region and the portion (16).

10. The method according to any of the claims 1 to 8, wherein the first magnetic field is acting in a region within the first container (10) and the microparticles (18) are exposed to the first and second magnetic fields by a permanent magnet (38) being respectively brought up to the region and the portion (16).

11. The method according to any of the preceding claims, wherein the microparticles (18) have an average diameter from 50 nm to 50 µm, preferably form 500 nm to 50 µm.

12. The method according to one of the preceding claims, wherein the microparticles (18) have a coating of glass, silicate, silane, an ion exchanger, a receptor, a ligand, an antigen, an antibody or a nucleic acid.

13. A device for carrying out a method a according one of the claims 1 to 12, comprising:
- a first container (10) for providing or receiving a first liquid (20) and paramagnetic microparticles (18),
- a first line (14) opening into the first container (10), and
- a portion (16) of the first line (14), which portion has an enlarged cross-sectional area in comparison with the remaining cross-sectional area of the first line (14),
- a first magnet (38) for producing a first magnetic field in a region of the first container (10) and in the portion (16), wherein the region, the portion (16), and the first magnet (38) are arranged such that a magnetic field, which is or can be created by the first magnet (38), is acting or capable of acting both in the region and in the portion (16), or
- a recess (36) for receiving a first magnet (38) for producing a first magnetic field in a region of the first container (10) and in the portion (16), wherein the region, the portion (16) and the recess (36) are arranged such that a magnetic field which is or may be generated by the first magnet (38) is acting or capable of acting both in the region as well as in the portion, wherein the region and the portion (16) are located on opposite sides of the recess (36), such that one pole of the first magnet (38) is capable of acting on the region and the other pole of the first magnet (38) is capable of acting on the portion, or
- a first magnet (38) for producing a first magnetic field in a region of the first container (10) and a second magnet for producing a second magnetic field in the portion (16),
- wherein the region, the portion (16) and the recess (36) or the first magnet (38) and, if present, the second magnet are disposed and/or arranged such that the magnetic field within the portion (16) has a higher average field strength than the magnetic field within the first container (10).

14. The device according the claim 13, wherein the magnet (38) is a permanent magnet.

15. Device according to claim 13 or 14, wherein the portion (16) is formed as a recess in the first line (14).

16. The device according to any of the claims 13 to 15, wherein the portion (16) is formed such that, when a liquid flows in a first direction, a laminar flow can be generated in the portion (16), and when the liquid flows in a second direction, in particular opposite to the first direction, a turbulent flow can be generated.

17. The device according to any of the claims 13 to 16, wherein at least one second line (34) is branching off from the first line (14).

18. The device according to any of the claims 13 to 17, wherein an opening of the second line (34) opens into the portion (16), wherein the opening is arranged such that liquid that is flowing through the opening into the portion (16) can cause turbulences in the portion (16) and, as a result, microparticles (18) deposited there can be suspended.

19. The device according to any of the claims 13 to 18, wherein the first line (14) has a diameter of 50 µm to 2 mm, preferably from 100 µm to 500 µm.

20. The device according to any of the claims 13 to 19, wherein the portion (16) has a cross-sectional area which is at most three times, preferably at most two times, as large as the cross-sectional area of the first line (14).

21. The device according to any of the claims 13 to 20, wherein the portion (16) has a cross-sectional area of at most 2 mm², preferably at most 1 mm².

22. The device as claimed according to any of the claims 13 to 21, wherein a second container (12) for the provision of a second liquid (40), a third container for the provision of a further liquid and/or a fourth container for receiving the first (20) and optionally second (40) and/or further liquid is provided in the device.

23. The device according to any of the claims 13 to 22, wherein the second line is opening into the second container (12), and optionally provided further lines (30) are opening into the third or fourth container.

24. The device according to any of the claims 13 to 23, wherein a plunger (22, 24) is provided in the first (10), second (12), third and/or fourth container, by means of which plunger the first (20), second (40) or further liquid can be moved.

25. The device according to any of the claims 13 to 24, wherein the first (10), second (12), third and/or fourth container in each case is provided in the form of an exchangeable cartridge (48, 50).

26. The device according to any of the claims 13 to 25, wherein the first (10), second (12), third and/or fourth container is formed cylindrically.

27. The device according to any of the claims 13 to 26, wherein the first (10), second (12), third or fourth container has a maximum volume of 50 µl to 50 ml, preferably from 500 µl to 5 ml.

28. The device according to any of the claims 13 to 27, wherein the device is insertable into an apparatus for sample processing, in particular automated sample processing.

29. The device according to claim 28, wherein the apparatus has at least one means for displacing the plunger (22, 24).

30. The device according to any of the claims 13 to 29, wherein the device is made of a plastic, in particular polycarbonate, preferably by means of an injection-molding process.

## Revendications

1. Procédé pour la purification améliorée d'une première substance, qui est liée à des microparticules paramagnétiques (18), les micros particules (18) étant en suspension dans un premier liquide (20), comportant les étapes suivantes :
a) dans un premier récipient (10) on expose les microparticules (18) à un premier champ magnétique, afin ainsi de les fixer et empêcher leur entraînement par un courant du premier liquide (20) et
b) au moins une partie du premier liquide (20) est passé, après l'étape a), dans une première direction, par un premier conduit (14), dans un segment (16) du premier conduit (14), et est exposée dans le segment (16) à un deuxième ou au premier champ magnétique, afin ainsi de fixer les microparticules (18) néanmoins entraînées, la surface de section transversale du premier conduit (14) étant agrandie dans le segment (16),
le deuxième ou premier champ magnétique présentant à l'intérieur du segment (16) une intensité moyenne de champ supérieure à celle du premier champ magnétique à l'intérieur du premier récipient (10).

2. Procédé selon la revendication 1, dans lequel après l'étape b) on fait passer un deuxième (40) et/ou autre liquide dans une deuxième direction dans le segment (16) et, dans une direction opposée à la première direction, dans le premier conduit (14), une action du premier champ magnétique et du deuxième champ magnétique éventuellement présent étant augmentée sur les microparticules (18), de sorte que les microparticules (18) maintenues dans le segment (16) sont mises en suspension et au moins partiellement renvoyées par entraînement vers les microparticules (18) maintenues dans l'étape a).

3. Procédé selon la revendication 2, dans lequel la deuxième direction est opposée à la première direction.

4. Procédé selon l'une quelconque des revendications précédentes, dans lequel le deuxième liquide (40) est choisi de manière que la première substance dans celui-ci reste liée aux microparticules (18) et d'autres substances ou d'autres impuretés se détachent des microparticules (18) ou de la première substance.

5. Procédé selon l'une quelconque des revendications précédentes, dans lequel l'autre liquide est choisi de manière que la première substance se détache dans celui-ci des microparticules (18).

6. Procédé selon l'une quelconque des revendications précédentes, dans lequel le segment (16) est formé de manière que, lors de l'écoulement du deuxième liquide (40) et/ou de l'autre liquide dans la première direction, il en résulte des tourbillonnements dans le segment, de sorte que les microparticules (18) qui y sont déposées sont mises en suspension.

7. Procédé selon l'une quelconque des revendications précédentes, dans lequel un deuxième conduit (34) comporte dans le segment (16) un orifice, par lequel on fait passer le deuxième liquide (40) et/ou autre liquide, de sorte qu'il en résulte des tourbillonnements dans le segment (16) et les microparticules (18) qui y sont déposées sont mises en suspension.

8. Procédé selon l'une quelconque des revendications précédentes, dans lequel les étapes a) et b) sont répétées avec le deuxième liquide (40) et/ou l'autre liquide au lieu du premier liquide (20).

9. Procédé selon l'une quelconque des revendications précédentes, dans lequel le premier champ magnétique agit dans une zone à l'intérieur du premier récipient (10) et on expose les microparticules (18) au premier champ magnétique en amenant un aimant permanent (38) sur la zone et le segment (16).

10. Procédé selon l'une quelconque des revendications 1 à 8, dans lequel le premier champ magnétique agit dans une zone à l'intérieur du premier récipient (10) et on expose les microparticules (18) au premier champ magnétique et au deuxième champ magnétique en amenant chaque fois un aimant permanent (38) au niveau de la zone et du segment (16).

11. Procédé selon l'une quelconque des revendications précédentes, dans lequel les microparticules (18) ont un diamètre moyen de 50 nm à 50 µm, de préférence de 500 nm à 50 µm.

12. Procédé selon l'une quelconque des revendications précédentes, dans lequel les microparticules présentent un revêtement à base de verre, de silicate, de silane, d'un échangeur ionique, d'un récepteur, d'un ligand, d'un antigène, d'un anticorps ou d'un acide nucléique.

13. Dispositif pour la mise en oeuvre d'un procédé selon l'une quelconque des revendications 1 à 12, comprenant :
- un premier récipient (10) destiné à fournir ou recevoir un premier liquide (20) et des microparticules paramagnétiques (18),
- un premier conduit (14) débouchant dans le premier récipient (10) et
- un segment (16) du premier conduit (14) qui présente une surface de section transversale agrandie par rapport à la surface de section transversale restante du premier conduit (14),
- un premier aimant (38) destiné à produire un premier champ magnétique dans une zone du premier récipient (10) et dans le segment (16), la zone, le segment (16) et le premier aimant (38) étant disposés de telle sorte qu'un champ magnétique produit ou pouvant être produit par le premier aimant (38) agit ou peut agir aussi bien dans la zone que dans le segment (16), ou
- un évidement (36) destiné à recevoir un premier aimant (38) pour la production d'un premier champ magnétique dans une zone du premier récipient (10) et dans le segment (16), la zone, le segment (16) et l'évidement (36) étant disposés de telle sorte qu'un champ magnétique produit ou pouvant être produit par le premier aimant (38) agit ou peut agir aussi bien dans la zone que dans le segment, la zone et le segment (16) se trouvant à des côtés opposés de l'évidement (36), de sorte qu'un pôle du premier aimant (38) peut agir sur la zone et l'autre pôle du premier aimant (38) peut agir sur le segment, ou
- un premier aimant (38) pour la production d'un premier champ magnétique dans une zone du premier récipient (10) et un deuxième aimant pour la production d'un deuxième champ magnétique dans le segment (16),
- la zone, le segment (16) et l'évidement (36) ou le premier aimant (38) et, s'il est présent, le deuxième aimant étant disposés et/ou formés de manière que le champ magnétique à l'intérieur du segment (16) présente une plus grande intensité moyenne de champ que le champ magnétique à l'intérieur du premier récipient (10).

14. Dispositif selon la revendication 13, dans lequel l'aimant (38) est un aimant permanent.

15. Dispositif selon la revendication 13 ou 14, dans lequel le segment (16) est constitué sous forme d'un évidement dans le premier conduit (14).

16. Dispositif selon l'une quelconque des revendications 13 à 15, dans lequel le segment (16) est formé de telle sorte que lors de l'écoulement d'un liquide dans une première direction dans le segment (16) il peut en résulter un flux laminaire et lors de l'écoulement du liquide dans une deuxième direction, en particulier opposée à la première direction, il peut en résulter un flux tourbillonnaire.

17. Dispositif selon l'une quelconque des revendications 13 à 16, dans lequel au moins un deuxième conduit (34) est dérivé du premier conduit (14).

18. Dispositif selon l'une quelconque des revendications 13 à 17, dans lequel un orifice du deuxième conduit (34) débouche dans le segment (16), l'orifice étant disposé de telle sorte qu'un liquide s'écoulant par l'orifice dans le segment (16) peut provoquer des tourbillonnements et que des microparticules (18) déposées dans celui-ci peuvent être mises en suspension.

19. Dispositif selon l'une quelconque des revendications 13 à 18, dans lequel le premier conduit (14) a un diamètre de 50 µm à 2 mm, de préférence de 100 µm à 500 µm.

20. Dispositif selon l'une quelconque des revendications 13 à 19, dans lequel le segment (16) présente une surface de section transversale qui est au maximum trois fois, de préférence au maximum deux fois plus grande que la surface de section transversale du premier conduit (14).

21. Dispositif selon l'une quelconque des revendications 13 à 20, dans lequel le segment (16) présente une surface de section transversale d'au maximum 2 mm², de préférence d'au maximum 1 mm².

22. Dispositif selon l'une quelconque des revendications 13 à 21, dans lequel est prévu un deuxième récipient (12) destiné à fournir un deuxième liquide (40), un troisième récipient destiné à fournir un autre liquide et/ou un quatrième récipient destiné à recevoir le premier liquide (20) et éventuellement le deuxième liquide (40) et/ou un autre liquide.

23. Dispositif selon l'une quelconque des revendications 13 à 22, dans lequel le deuxième conduit (34) débouche dans le deuxième récipient (12) et d'autres conduits (30) éventuellement présents débouchent dans le troisième ou quatrième récipient.

24. Dispositif selon l'une quelconque des revendications 13 à 23, dans lequel dans chacun des premier (10), deuxième (12), troisième et/ou quatrième récipients est disposé un piston (22, 24) coulissant dans celui-ci, au moyen duquel peut être déplacé le premier liquide (20), le deuxième liquide (40) ou un autre liquide.

25. Dispositif selon l'une quelconque des revendications 13 à 24, dans lequel chacun des premier (10), deuxième (12), troisième et/ou quatrième récipients est prévu sous forme d'une cartouche échangeable (48, 50).

26. Dispositif selon l'une quelconque des revendications 13 à 25, dans lequel chacun des premier (10), deuxième (12), troisième et/ou quatrième récipients a une forme cylindrique.

27. Dispositif selon l'une quelconque des revendications 13 à 26, dans lequel chacun des premier (10), deuxième (12), troisième et/ou quatrième récipients présente un volume maximum de 50 µl à 50 ml, de préférence de 500 µl à 5 ml.

28. Dispositif selon l'une quelconque des revendications 13 à 27, le dispositif étant utilisable dans un appareil destiné au traitement d'échantillons, en particulier automatisé.

29. Dispositif selon la revendication 28, dans lequel l'appareil comporte au moins un organe destiné au déplacement du piston (22, 24).

30. Dispositif selon l'une quelconque des revendications 13 à 29, le dispositif étant fabriqué à base d'une matière plastique, en particulier de polycarbonate, de préférence au moyen d'un procédé de moulage par injection.
